# EUROPEAN PATENT APPLICATION

(11) **EP 1 849 761 A1**
(43) Date of publication of application: **31.10.2007**
(21) Application number: 06713353.8
(22) Date of filing: 02.02.2006
(51) Int. Cl.: C07C 45/64, C07C 49/255, C07D 239/26, C07D 239/38, C07D 239/42

(54) **PROCESS FOR PRODUCTION OF 4-ACETYLPYRIMIDINES AND CRYSTALS THEREOF**

(30) Priority: 02.02.2005 JP 2005027100
(71) Applicant: Ajinomoto Co., Inc., Tokyo 104-8315 (JP)
(72) Inventor: TAKAHASHI, Daisuke c/o AJINOMOTO CO., INC., Kanagawa, 2108681 (JP); IZAWA, Kunisuke c/o AJINOMOTO CO., INC., Kanagawa, 2108681 (JP)
(74) Representative: Nicholls, Kathryn Margaret
(86) International application number: PCT/JP2006/302210
(87) International publication number: WO 2006/083010

(57) **Abstract**

Compound (II) is reacted with formate in the presence of an alkali metal alkoxide and the like to give compound (III); then this is reacted with compound (IVa) or compound (IVb) to give compound (V); then this is reacted with compound (VI) to give compound (VII); and then this is deprotected to give compound (I).

The present invention provides an industrially advantageous production method of a 4-acetylpyrimidine compound useful as a synthetic intermediate for a pharmaceutical product: 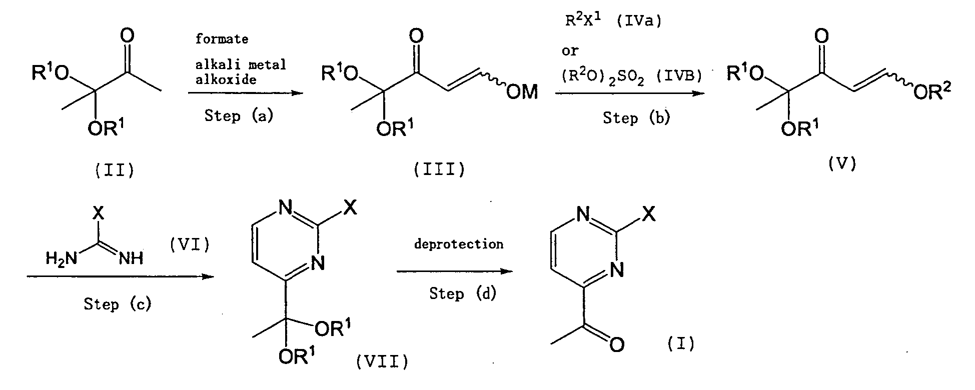 wherein X is a methylthio group and the like, R¹ is a lower alkyl group and the like, R² is a lower alkyl group optionally having substituent(s) and the like, M is an alkali metal and X¹ is a halogen atom.

## Description

### Technical Field

The present invention relates to a production method of 4-acetylpyrimidine compound useful as an intermediate for synthesizing a pharmaceutical product, and a novel crystal thereof.

### Background Art

4-Acetylpyrimidine compound represented by the formula (I) : 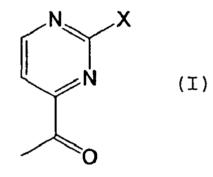 wherein X is a hydrogen atom, a lower alkyl group optionally having substituent(s), an aryl group optionally having substituent(s), an aralkyl group optionally having substituent(s), -OR³, -SR³ or-NR³R⁴ (wherein R³ and R⁴ are the same or different and each is a hydrogen atom, a lower alkyl group optionally having substituent(s), an aralkyl group optionally having substituent(s) or an aryl group optionally having substituent(s), or R³ and R⁴ may form, together with the adjacent nitrogen atom, an aliphatic hetero ring optionally having substituent(s)),
or a salt thereof, is useful as an intermediates for synthesizing various pharmaceutical products such as a MAP kinase inhibitor, a tyrosine kinase inhibitor and the like (WO03/035638 and WO03/011838).

As the production method of the 4-acetylpyrimidine compound, a method comprising coupling 4-chloro-2-methylthiopyrimidine (1) and tributyl(1-ethoxyvinyl)tin (2) in the presence of a palladium catalyst to give enol ether (3), and subjecting the compound to hydrolysis is described (WO03/035638 and Journal of heterocyclic Chemistry, 1985, vol. 22, p.1723) as shown in the following scheme.

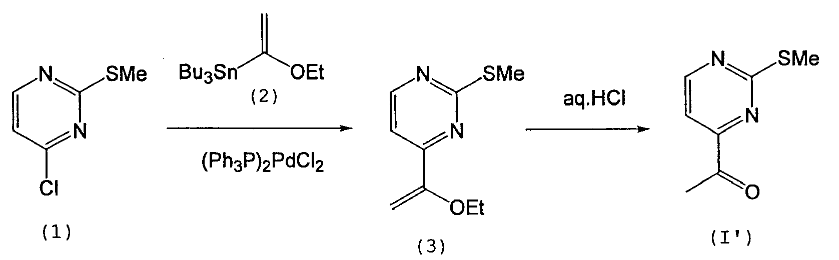

However, this method requires use of an expensive palladium catalyst and an expensive tin reagent, tin reagent (2) has strong toxicity, and the method is associated with problems of safety and hygiene. Accordingly, this method is not entirely an industrially advantageous method.

As other methods, a method comprising reacting 3,3-dimethoxy-2-butanone (4) with N,N-dimethylformamide dimethylacetal (5) to give enamine (6), reacting the compound with thiourea and methyl iodide to give 4-acetylpyrimidine dimethylacetal (7) and subjecting the compound to hydrolysis is described (WO03/035638 and Chemical & Pharmaceutical Bulletin, 2003, vol.51, p.975), as shown in the following scheme:

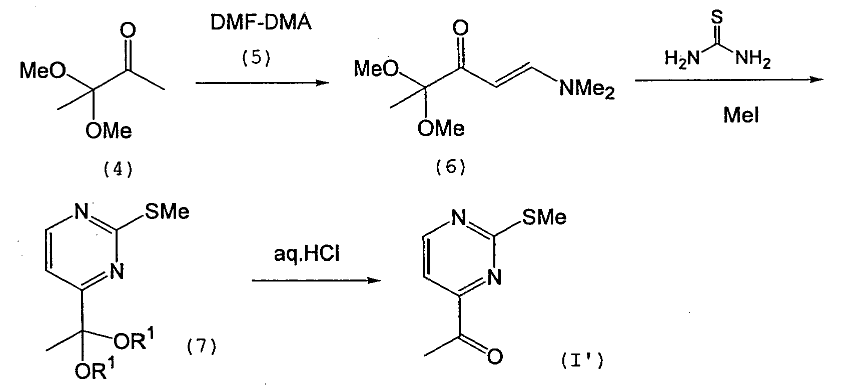

In this method, however, since N,N-dimethylformamide dimethylacetal and methyl iodide to be used are expensive, the cost becomes high, and problems of coloring, inhibition of crystallization and the like due to the influence of impurity in the reaction between 3,3-dimethoxy-2-butanone and N,N-dimethylformamide,dimethylacetal occur.

As other method, a method comprising condensing pyrimidine-4-carboxylic acid compound (8) with N,O-dimethylhydroxylamine to give Wein-reb amide (9), and reacting the compound with a Grignard reagent is described (WO03/011838), as shown in the following scheme:

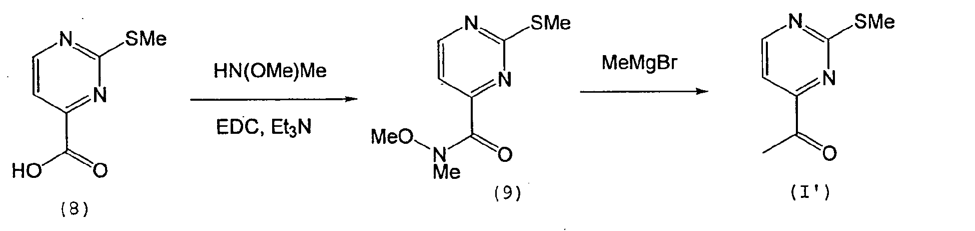

In this method, however, the cost becomes high since N,O-dimethylhydroxylamine and 1-(3-dimethylaminopropyl)-3-ethylcarbodiimide hydrochloride (EDC·HCl) used as a condensing agent are expensive, and the yield is not satisfactory.

In the above-mentioned publications, 1-(2-Methylsulfanylpyrimidin-4-yl)ethanone, which is a representative compound of the 4-acetylpyrimidine compound, was isolated as an oil, or is not described as having been obtained as a crystal. In consideration of easy quality management as an intermediate for a pharmaceutical product and the possibility of distribution as a synthetic intermediate, isolation as a crystal is preferable.

An object of the present invention is provision of an industrially advantageous production method of a 4-acetylpyrimidine compound useful as a synthetic intermediate for a pharmaceutical product.

### Disclosure of the Invention

The present inventors have conducted intensive studies in an attempt to solve the above-mentioned problems and established a synthetic route permitting production of a 4-acetylpyrimidine compound at a low cost and in a high yield. In addition, they have first succeeded in obtaining 1-(2-methylsulfanylpyrimidin-4-yl)ethanone as a crystal by studying the crystallization conditions, which resulted in the completion of the present invention. Accordingly, the present invention provides the following [1] - [13].
[1] A production method of a compound represented by the formula (III): 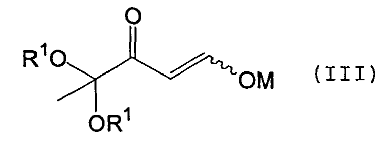 wherein R¹ is a lower alkyl group, or optionally bonded to each other to form an alkylene group optionally having substituent(s), M is an alkali metal and a wavy line is a trans-isomer, a cis-isomer or a mixture thereof (hereinafter sometimes to be referred to as compound (III)), which comprises reacting a compound represented by the formula (II): 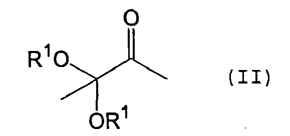 wherein R¹ is as defined above (hereinafter sometimes to be referred to as compound (II)) with a formate in the presence of an alkali metal alkoxide or an alkali metal hydride.
[2] A production method of a compound represented by the formula (V) : 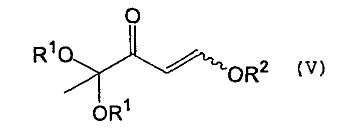 wherein R² is a lower alkyl group optionally having substituent(s) or an aralkyl group optionally having substituent(s) and R¹ and a wavy line are as defined above (hereinafter sometimes to be referred to as compound (V)), which comprises reacting compound (III) with a compound represented by the formula (IVa): R²X¹ or the formula (IVb): (R²O)₂SO₂ (wherein X¹ is a halogen atom, and R² is as defined above) (hereinafter sometimes to be referred to as compound (IVa) and compound (IVb), respectively).
[3] A production method of a compound represented by the formula (VII) : 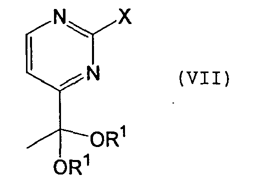 wherein X is a hydrogen atom, a lower alkyl group optionally having substituent(s), an aryl group optionally having substituent(s), an aralkyl group optionally having substituent(s), -OR³, -SR³ or -NR³R⁴ (wherein R³ and R⁴ are the same or different and each is a hydrogen atom, a lower alkyl group optionally having substituent(s), an aralkyl group optionally having substituent(s) or an aryl group optionally having substituent(s), or R³ and R⁴ optionally form, together with the adjacent nitrogen atom, aliphatic hetero ring optionally having substituent(s), and R¹ is as defined above (hereinafter sometimes to be referred to as compound (VII)), which comprises reacting compound (V) with a compound represented by the formula (VI): 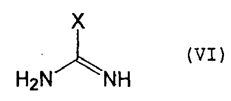 wherein X is as defined above (hereinafter sometimes to be referred to as compound (VI)) or a salt thereof.
[4] A production method of compound (VII) or a salt thereof, which comprises the following steps (a) to (c);
   (a) reacting compound (II) with a formate in the presence of an alkali metal alkoxide or an alkali metal hydride to give compound (III);
   (b) reacting the obtained compound (III) with compound (IVa) or compound (IVb) to give compound (V); and
   (c) reacting the obtained compound (V) with compound (VI) or a salt thereof to give compound (VII).
[5] A production method of a compound represented by the formula (I) 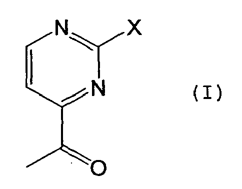 wherein X is as defined above (hereinafter sometimes to be referred to as compound (I)) or a salt thereof, which comprises deprotecting the compound (VII) obtained by the method of the above-mentioned [3] or [4].
[6] The production method of any of the above-mentioned [3] to [5], wherein X is a methyl group, a methylthio group, a methoxy group, a methylamino group or a phenyl group.
[7] The production method of any of the above-mentioned [1] to [5], wherein R¹ is a methyl group or an ethyl group.
[8] The production method of any of the above-mentioned [2] to [4], wherein R² is a methyl group, an ethyl group, an n-propyl group, an n-butyl group or a benzyl group.
[9] A compound represented by the formula (V'): 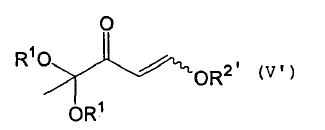 wherein R¹ is lower alkyl group, or may be bonded to each other to form an alkylene group optionally having substituent(s), R²' is an alkali metal, a lower alkyl group optionally having substituent(s) or an aralkyl group optionally having substituent(s), and a wavy line is a trans-isomer, a cis-isomer or a mixture thereof.
[10] A production method of a crystal of 1-(2-methylsulfanylpyrimidin-4-yl)ethanone, which comprises crystallization from a mixed solvent of ethyl acetate and at least one solvent selected from a hydrocarbon solvent.
[11] The production method of the above-mentioned [10], wherein the crystallization is performed using a mixed solvent of hexane and ethyl acetate.
[12] A production method of a crystal of 1-(2-methylsulfanylpyrimidin-4-yl)ethanone, which comprises crystallization from a mixed solvent of water and at least one solvent selected from acetone, methanol, ethanol and acetonitrile.
[13] The production method of the above-mentioned [12], wherein the crystallization is performed using a mixed solvent of acetone and water.
[14] A crystal of 1-(2-methylsulfanylpyrimidin-4-yl)ethanone.
[15] A crystal of 1-(2-methylsulfanylpyrimidin-4-yl)ethanone, showing a powder X-ray crystal diffraction pattern having characteristic peaks at diffraction angles (2θ±0.1°) of about 12.3°, about 12.6°, about 17.4°, about 24.7° and about 26.5°.
[16] The crystal of the above-mentioned [15], which is obtained by crystallization from a mixed solvent of hexane and ethyl acetate.
[17] A crystal of 1-(2-methylsulfanylpyrimidin-4-yl)ethanone, showing a powder X-ray crystal diffraction pattern having characteristic peaks at diffraction angles (2θ±0.1°) of about 13.7°, about 14.8°, about 17.9°, about 21.2° an d about 36.1°.
[18] A crystal of the above-mentioned [17], which is obtained by crystallization from a mixed solvent of acetone and water.

### Brief Description of the Drawings

Fig. 1 shows a powder X-ray crystal diffraction pattern of 1-(2-methylsulfanylpyrimidin-4-yl)ethanone (Example 12).
Fig. 2 shows a powder X-ray crystal diffraction pattern of 1-(2-methylsulfanylpyrimidin-4-yl)ethanone (Example 13).

### Detailed Description of the Invention

The present invention is explained in detail in the following.

### 1. Explanation of symbols

The "lower alkyl group" for R¹ is a linear or branched chain alkyl preferably having 1 - 5, more preferably 1 or 2, carbon atoms and, for example, methyl group, ethyl group, n-propyl group, isopropyl group, n-butyl group, isobutyl group, sec-butyl group and the like can be mentioned. Of these, methyl group and ethyl group are preferable.

The "lower alkyl group optionally having substituent(s)" for X, R², R^{2'}, R³ or R⁴ is the above-mentioned alkyl group optionally substituted by one or more of the following substituents, and when the number of the substituents is two or more, they may be the same or different. Examples of the substituent include nitro group, linear or branched chain alkoxy group (preferable carbon atoms: 1 - 6, e.g., methoxy group and the like), halogen atom (e.g., chlorine atom, fluorine atom and the like), hydroxyl group and the like.

As the alkylene group of the alkylene group optionally having substituent(s), which is formed by R¹ bonded to each other, for example, ethylene group, trimethylene group, tetramethylene group and the like can be mentioned, with preference given to ethylene group and trimethylene group. The alkylene group may be substituted by one or more substituents mentioned below. When the number of the substituents is two or more, they may be the same or different. Examples of the substituent include nitro group, linear or branched chain alkoxy group (preferable carbon atoms: 1 - 6, e.g., methoxy group and the like), halogen atom (e.g., chlorine atom, fluorine atom and the like), linear or branched chain alkyl group (preferable carbon atoms: 1 - 4, e.g., methyl group, ethyl group, propyl group and the like), hydroxyl group and the like.

The "aryl group" of the "aryl group optionally having substituent(s)" for R³, R⁴ or X is an aryl group having preferably 6 - 10, more preferably 6, carbon atoms and, for example, phenyl group, 1-naphthyl group, 2-naphthyl group and the like can be mentioned. The aryl group may be substituted by one or more substituents mentioned below. When the number of the substituents is two or more, they may be the same or different. Examples of the substituent include nitro group, linear or branched chain alkoxy group (preferable carbon atoms: 1 - 6, e.g., methoxy group and the like), halogen atom (e.g., chlorine atom, fluorine atom and the like), linear or branched chain alkyl group (preferable carbon atoms: 1 - 4, e.g., methyl group, ethyl group, propyl group and the like), hydroxyl group and the like.

The "aralkyl group" of the "aralkyl group optionally having substituent(s)" for X, R², R^{2'}, R³ or R⁴ is aralkyl group wherein the aryl moiety is aryl group preferably having 6 - 10, more preferably 6, carbon atoms and the alkyl moiety is linear or branched chain alkyl group preferably having 1 - 6 carbon atoms. For example, benzyl group and the like can be mentioned.

The aralkyl group may be substituted by one or more substituents mentioned below. When the number of the substituents is two or more, they may be the same or different. Examples of the substituent include nitro group, linear or branched chain alkoxy group (preferable carbon atoms: 1 - 6, e.g., methoxy group and the like), halogen atom (e.g., chlorine atom, fluorine atom and the like), linear or branched chain alkyl group (preferable carbon atoms: 1 - 4, e.g., methyl group, ethyl group, propyl group and the like), hydroxyl group and the like.

As the "halogen atom" for X¹, chlorine atom, bromine atom, iodine atom can be mentioned, with preference given to chlorine atom and bromine atom.

Examples of the "aliphatic hetero ring" optionally formed by R³ and R⁴ together with the adjacent nitrogen atom include 5-or 6-membered aliphatic hetero ring containing a carbon atom and at least one nitrogen atom and optionally containing 1 - 3 hetero atoms selected from oxygen atom, sulfur atom and nitrogen atom, such as pyrrolidine, piperidine, morpholine, thiomorpholine, piperazine and the like.

The aliphatic hetero ring may be substituted by one or more substituents mentioned below. When the number of the substituents is two or more, they may be the same or different. Examples of the substituent include nitro group, linear or branched chain alkoxy group (preferable carbon atoms: 1 - 6, e.g., methoxy group and the like), halogen atom (e.g., chlorine atom, fluorine atom and the like), linear or branched chain alkyl group (preferable carbon atoms: 1 - 4, e.g., methyl group, ethyl group, propyl group and the like), hydroxyl group and the like.

As the alkali metal for R²' or M, lithium, sodium, potassium and the like can be mentioned.

Now, preferable embodiments of each symbol are explained below.

As the X, methyl group, methylthio group, methoxy group, methylamino group or phenyl group is preferable, and methylthio group or phenyl group is more preferable.

As the R¹, methyl group, ethyl group, n-propyl group or n-butyl group is preferable, and methyl group or ethyl group is more preferable.

As the R², methyl group, ethyl group, n-propyl group, n-butyl group or benzyl group is preferable, methyl group or ethyl group is more preferable.

M is preferably sodium or potassium.

Since compound (I) has a pyrimidine ring, depending on the kind of the substituent for X, it can form a salt such as an inorganic acid salt (e.g., hydrochloride, sulfate and the like), an organic acid salt (e.g., acetate, trifluoroacetate, tosylate, mesylate and the like) and the like.

As compound (VI), those commercially available in the form of a salt such as an inorganic acid salt (e.g., hydrochloride, sulfate and the like), an organic acid salt (e.g., acetate, trifluoroacetate, tosylate, mesylate and the like) and the like can generally be used.

### 2. Production method of the compound of the present invention

The production method of the present invention is represented by the following reaction scheme. 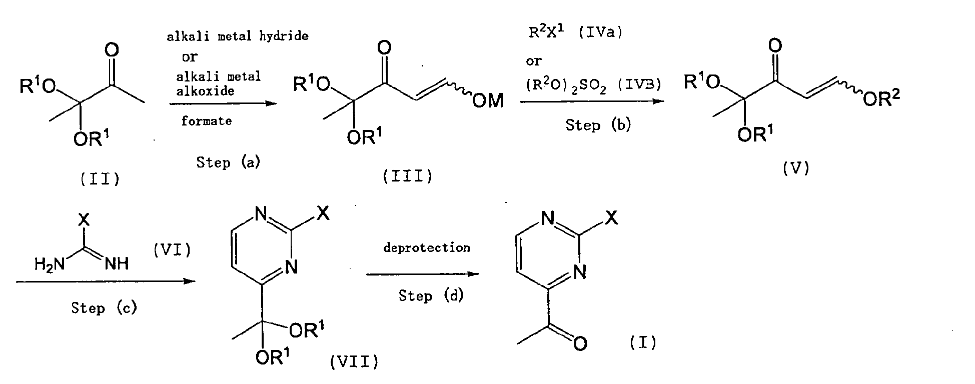 wherein each symbol and the wavy line are as defined above.

Namely, the present invention is characterized in that it contains at least one of the steps (a) - (c) shown by the above-mentioned reaction scheme.

By employing such synthetic route, particularly steps (a) and (b), pyrimidine ring can be constructed in a high yield without using an expensive reagent such as N,N-dimethylformamide dimethylacetal, and without coloring and the like.

Steps (a) - (d) are explained below.

### 2-1. Step (a)

Step (a) can be performed, for example, by mixing compound (II), alkali metal alkoxide or alkali metal hydride, and formate in a solvent. The order of addition of each reagent is not particularly limited and they can be successively or simultaneously added. Preferably, compound (II) and formate are added to a mixture of alkali metal alkoxide or alkali metal hydride and a solvent.

Examples of the alkali metal alkoxide used in step (a) include sodium methoxide, sodium ethoxide, sodium tert-butoxide, potassium methoxide, potassium ethoxide, potassium tert-butoxide and the like. Examples of the alkali metal hydride include sodium hydride, potassium hydride and lithium hydride. Preferred are sodium methoxide, potassium tert-butoxide or sodium hydride. The amount of alkali metal alkoxide or alkali metal hydride to be used is generally 0.9 - 1.5 equivalents, preferably 1 - 1.1 equivalents, relative to compound (II).

Examples of the formate used in step (a) include methyl formate, ethyl formate, butyl formate and the like, with preference given to ethyl formate and methyl formate. The amount of formate to be used is generally 0.8 - 5 equivalents, preferably 1 or 2 equivalents, relative to compound (II).

The solvent usable for step (a) may be any as long as it does not inhibit the reaction and, for example, ethers (e.g., diethyl ether, tetrahydrofuran (THF), diisopropyl ether and the like) and the like can be mentioned, which may be used alone or in a combination of two or more kinds thereof. Preferred are diethyl ether and THF. The amount of the solvent to be used is 5-fold weight to 50-fold weight, preferably 8-fold weight to 20-fold weight, relative to compound (II).

Step (a) is generally performed within the range of from - 50°C to the reflux temperature of the solvent to be used (preferably -10°C to 40°C). The reaction time is generally 1 hr - 50 hr (preferably 3 hr - 24 hr) within the above-mentioned temperature range.

After completion of the reaction of step (a), compound (III) is present in the form of an alkali metal salt.

When compound (III) is to be isolated, general isolation and purification methods, for example, concentration or crystallization of the reaction mixture enables isolation of compound (III). It is also possible to subject compound (III) to the next step without particular isolation and purification.

Compound (II), which is a starting material of step (a), can be produced by a known method, or a commercially available product can also be used.

### 2-2. Step (b)

Step (b) can be performed, for example, by mixing compound (III) with compound (IVa) or compound (IVb) in a solvent. The order of addition of respective reagents is not particularly limited and they can be added successively or simultaneously.

Examples of the compound (IVa) used in step (b) include benzyl bromide, butyl bromide and the like, with preference given to benzyl bromide. Examples of the compound (IVb) include methyl sulfate, ethyl sulfate and the like, with preference given to methyl sulfate. The amount of compound (IVa) or compound (IVb) to be used is generally 0.8 - 3 equivalents, preferably 1 - 1.5 equivalents, relative to compound (III).

The solvent usable for step (b) may be any as long as it does not inhibit the reaction and, for example, esters (e.g., ethyl acetate, isopropyl acetate, butyl acetate and the like), nitriles (e.g., acetonitrile and the like), ethers (THF, diisopropyl ether and the like) and the like can be mentioned, which may be used alone or in a combination of two or more kinds thereof, with preference given to acetonitrile, ethyl acetate and butyl acetate. The amount of the solvent to be used is 5-fold weight to 50-fold weight, preferably 8-fold weight to 20-fold weight, relative to compound (III).

Step (b) is generally performed within the range of from 10°C to the reflux temperature of the solvent to be used (preferably 40°C - 120°C). The reaction time is generally 0.5 hr - 30 hr (preferably 3 hr - 24 hr) within the above-mentioned temperature range.

Compound (V) obtained in step (b) can be isolated and purified by a conventional method. For example, compound (V) can be isolated by, after completion of the reaction, where necessary, extracting with ethyl acetate etc., washing with water, aqueous acidic solution (e.g., hydrochloric acid, sulfuric acid and the like) or aqueous alkali solution (e.g., saturated aqueous sodium hydrogen carbonate, brine and the like) and the like, and concentrating the organic layer obtained by partitioning. Moreover, compound (V) can be purified by, but is not limited to, performing distillation or silica gel column chromatography. In addition, compound (V) can be subjected to the next step without purification.

### 2-3. Step (c)

Step (c) can be performed, for example, by mixing compound (V) and compound (VI) in a solvent. The order of addition of respective reagents is not particularly limited and they can be added successively or simultaneously.

Compound (VI) to be used for step (c) is generally commercially available in the form of a stable salt. Examples of the salt of the compound (VI) include an acid addition salt such as hydrochloride, sulfate, acetate and the like.

In step (c), when an acid addition salt of compound (VI) is to be used, it can be neutralized with a base in a solvent. For example, the salt can be once converted to a free form, followed by reaction with compound (V), or a salt of compound (VI) and compound (V) can be dissolved in a solvent, followed by addition of a base to allow reaction. The base to be used for neutralization is not particularly limited and, for example, sodium hydroxide, potassium hydroxide, sodium carbonate, potassium carbonate, triethylamine, sodium methoxide, sodium ethoxide and the like can be mentioned. The amount of the base to be used is not particularly limited as long as it can convert a salt of compound (VI) to a free form. From the economical view, it is generally 0.8 - 3 equivalents, preferably 1 - 1.5 equivalents, relative to a salt of compound (VI).

The solvent usable for step (c) may be any as long as it does not inhibit the reaction and, for example, esters (e.g., ethyl acetate, isopropyl acetate, butyl acetate and the like), nitriles (e.g., acetonitrile and the like), alcohols (e.g., methanol, ethanol, isopropyl alcohol and the like) and the like can be mentioned, which may be used alone or in a combination of two or more kinds thereof, acetonitrile, butyl acetate, ethyl acetate are preferable. The amount of the solvent to be used is 5-fold weight to 50-fold weight, preferably 8-fold weight to 20-fold weight, relative to compound (V).

Step (c) is generally performed within the temperature range of from 20°C to the reflux temperature of the solvent to be used (preferably 40 - 110°C). The reaction temperature is generally 1 hr - 30 hr (preferably 3 hr - 24 hr) within the above-mentioned temperature range.

Compound (VII) obtained in step (c) can be isolated and purified by a conventional method. For example, compound (VII) can be isolated by, after completion of the reaction, where necessary, extracting with ethyl acetate etc., washing with water, aqueous acidic solution (e.g., hydrochloric acid, sulfuric acid and the like) or aqueous alkali solution (e.g., saturated aqueous sodium hydrogen carbonate, brine and the like) and the like, and concentrating the organic layer obtained by partitioning. Moreover, compound (VII) can be purified by, but is not limited to, performing crystallization by adding a crystallization solvent (e.g., ethers (e.g., diethyl ether, THF and the like), acetone, acetonitrile, hydrocarbon solvent (e.g., toluene, benzene, hexane, heptane and the like), a halogen solvent (e.g., dichloromethane, dichloroethane and the like), alcohols (e.g., methanol, ethanol, isopropanol and the like), water, a mixed solvent thereof and the like), or silica gel column chromatography. In addition, compound (VII) can be subjected to the next step without purification.

The compound represented by the formula (V'): 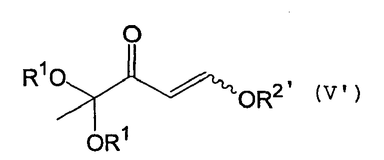 wherein R¹ is a lower alkyl group or optionally bonded to each other to form an alkylene group optionally having substituent(s), R²' is an alkali metal, a lower alkyl group optionally having substituent(s) or an aralkyl group optionally having substituent(s), and the wavy line is a trans-isomer, a cis-isomer or a mixture thereof), which is produced by step (b) or (c), is a novel compound.

### 2-4. Step (d)

For step (d), various methods known per se capable of deprotecting the acetal group of compound (VII) and converting the compound to ketone can be applied. For example, deprotection method by acid hydrolysis can be mentioned. While deprotection by acid hydrolysis is explained in the following, step (d) is not limited thereto.

Acid hydrolysis can be performed, for example, by mixing compound (VII) and acid in a solvent and water. The order of addition is not particularly limited and they can be added successively or simultaneously.

Examples of the acid include hydrochloric acid, sulfuric acid, acetic acid, trichloroacetic acid and the like, with preference given to hydrochloric acid. The amount of the acid to be used is generally 0.1 - 20 equivalents, preferably 0.5 - 3 equivalents, relative to compound (VII).

The solvent used for acid hydrolysis may be any as long as it does not inhibit this reaction and, for example, esters (e.g., ethyl acetate, isopropyl acetate, butyl acetate and the like), alcohols (e.g., methanol, ethanol, isopropyl alcohol and the like), ketones (e.g., acetone, methyl isobutyl ketone and the like) or a mixed solvent of these and water and the like can be mentioned, which may be used alone or in a combination of two or more kinds thereof, with preference given to ethyl acetate, methanol and a mixed solvent of methanol and water. The amount of the solvent to be used is 3-fold weight to 50-fold weight, preferably 5-fold weight to 20-fold weight, relative to compound (VII).

The acid hydrolysis is generally performed within the temperature range of 0°C to the reflux temperature of the solvent to be used (preferably 0 - 30°C). The reaction time is generally 0.1 hr - 30 hr (preferably 0.5 hr - 3 hr) within the above-mentioned temperature range.

Compound (I) obtained in step (d) can be isolated and purified by a conventional method. For example, compound (I) can be isolated by, after completion of the reaction, where necessary, extracting with ethyl acetate etc., washing with water, aqueous acidic solution (e.g., hydrochloric acid, sulfuric acid and the like) or aqueous alkali solution (e.g., saturated aqueous sodium hydrogen carbonate, brine and the like) and the like, and concentrating the organic layer obtained by partitioning. Moreover, compound (I) can be purified by, but is not limited to, performing crystallization by adding a crystallization solvent (e.g., ethers (e.g., diethyl ether, THF and the like), acetone, acetonitrile, hydrocarbon solvent (e.g., toluene, benzene, hexane, heptane and the like), a halogen solvent (e.g., dichloromethane, dichloroethane and the like), alcohols (e.g., methanol, ethanol, isopropanol and the like), water, a mixed solvent thereof and the like), or silica gel column chromatography. In addition, compound (I) can be used as an intermediate without purification.

### 3. Crystal of 1-(2-methylsulfanylpyrimidin-4-yl)ethanone and production method thereof

The crystal of 1-(2-methylsulfanylpyrimidin-4-yl)ethanone can be obtained by crystallizing the purified product or crude product obtained in the above-mentioned step (d) in a solvent.

Examples of the solvent to be used for crystallization include a mixed solvent of at least one solvent selected from hydrocarbon solvents such as hexane, heptane and the like and ethyl acetate and a mixed solvent of at least one solvent selected from acetone, methanol, ethanol and acetonitrile and water, with preference given to a mixed solvent of hexane and ethyl acetate or a mixed solvent of acetone and water.

In the case of a mixed solvent of ethyl acetate and a hydrocarbon solvent, the mixing ratio thereof (ethyl acetate:hydrocarbon solvent) is generally.1:50 - 3:10 (v/v), preferably 1:10 (v/v).

In the case of a mixed solvent of at least solvent selected from acetone, methanol, ethanol and acetonitrile and water, the mixing ratio thereof is generally 1:20 - 3:1 (v/v), preferably 1:8 (v/v).

The amount of the solvent to be used is 5 ml - 50 ml, preferably 8 ml - 20 ml, per 1 g of 1-(2-methylsulfanylpyrimidin-4-yl)ethanone.

When a mixed solvent is used for crystallization, 1-(2-methylsulfanylpyrimidin-4-yl)ethanone may be dissolved in a solvent mixed in advance. However, it is preferable to dissolve 1-(2-methylsulfanylpyrimidin-4-yl)ethanone in a good solvent (e.g., ethyl acetate, acetone and the like) and then add a poor solvent (e.g., hexane and water) to perform crystallization.

Crystallization is generally performed within the range of -20°C to 20°C, preferably -10 to 10°C, for about 1 hr - 30 hr.

The precipitated crystals can be obtained by a solid-liquid separation method known per se such as filtration, centrifugation and the like. The obtained crystals are desirably washed with the crystallization solvent used.

The thus-obtained crystals of 1-(2-methylsulfanylpyrimidin-4-yl)ethanone can be in plural polymorphic crystal forms depending on the crystallization conditions such as the solvent used and the like, and have specific properties.

For example, the crystals obtained by crystallization from a mixed solvent of hexane and ethyl acetate show a diffraction pattern having characteristic peaks at diffraction angles (2θ±0.1°) of about 12.3°, about 12.6°, about 17.4°, about 24.7° and about 26.5°, and the crystals obtained by crystallization from a mixed solvent of acetone and water show a diffraction pattern having characteristic peaks at diffraction angles (2θ±0.1°) of about 13.7°, about 14.8°, about 17.9°, about 21.2° and about 36.1°, both by powder X-ray crystal diffraction (measurement conditions are as shown in the Examples).

### Examples

The present invention is explained in more detail in the following by referring to Examples, which are not to be construed as limitative.

### Example 1: 1-hydroxy-4,4-dimethoxy-pent-1-en-3-one-sodium salt

Under nitrogen atmosphere, sodium methoxide (8.17 g, 151 mmol) was suspended in diethyl ether (200 mL), a mixture of 3,3-dimethoxy-butan-2-one (20.0 g, 151 mmol) and ethyl formate (14.1 g, 190 mmol) was added dropwise over 1 hr under ice-cooling, and the mixture was stirred at room temperature overnight. After the reaction, the precipitate was filtrated and dried under reduced pressure to give the title compound (25.7 g, 141 mmol). ¹H-NMR(DMSO-d₆) δ 1.85(3H, s), 3.04(6H, s), 4.97(1H, d, J=9.6Hz), 9.24(1H, d, J=9.6Hz)

### Example 2: 1,4,4-trimethoxy-pent-1-en-3-one

1-Hydroxy-4,4-dimethoxy-1-pentan-3-one-sodium salt (1.20 g, 0.66 mmol) was dissolved in acetonitrile (15 ml), methyl sulfate (0.61 mL, 0.68 mmol) was added thereto, and the mixture was stirred at 80°C overnight. The reaction mixture was concentrated under reduced pressure, and ethyl acetate was added to the residue. The obtained mixture was washed successively with water and saturated brine, and the solvent was evaporated. The residue was purified by silica gel chromatography to give the title compound (1.06 g, 0.61 mmol).
¹H-NMR(CDCl₃) δ 1.40(3H, s), 3.26(6H, s), 3.76(3H, s), 6. 07 (1H, d, J=12.5Hz), 7.77(1H, d, J=12.5Hz)

### Example 3: 1,4,4-trimethoxy-pent-1-en-3-one

1-Hydroxy-4,4-dimethoxy-pent-l-en-3-one-sodium salt (100 mg, 0.55 mmol) was dissolved in butyl acetate (1.5 ml), methyl sulfate (522 µg, 0.55 mmol) was added thereto, and the mixture was stirred at 100°C for 3 hr. The reaction mixture was analyzed by HPLC and production of the title compound (91 mg, 0.53 mmol) was confirmed.
HPLC analysis conditions:
column: Inertsil ODS-2 4.6 mm×150φ
column temperature: 40°C
detection wavelength: 254 nm
mobile phase: Buffer (NaH₂PO₄, pH 4.5):CH₃CN=50:50
flow rate: 0.8 ml/min.

### Example 4: 1-ethoxy-4,4-dimethoxy-pent-1-en-3-one

In the same manner as in Example 2 except that ethyl sulfate was used instead of methyl sulfate, the title compound was obtained.
¹H-NMR(CDCl₃,) δ 1.36(3H, t, J=7.1Hz), 3.26(6H, s), 4.00(2H, q, J=7.1Hz), 6.07(1H, d, J=12.5Hz), 7.73(1H, d, J=12.5Hz)

### Example 5: 1-benzyloxy-4,4-dimethoxy-pent-1-en-3-one

In the same manner as in Example 2 except that benzyl bromide was used instead of methyl sulfate, the title compound was obtained.
¹H-NMR(CDCl₃) δ 1.39(3H, s), 3.26(6H, s), 5.09(2H, s), 6.18 (1H, d, J=12.5), 7.12-7.38(5H, m), 7.84-7.81(1H, d, J=12.5)

### Example 6: 4-(1,1-dimethoxy-ethyl)-2-methylsulfanyl-pyrim.idine

Methylisothiourea sulfate (880 mg, 3.16 mmol) and sodium carbonate (680 mg, 6.41 mmol) were stirred in acetonitrile (10 ml) for 30 min, 1,4,4-trimethoxy-pent-l-en-3-one (1.0 g, 5.74 mmol) was added thereto, and the mixture was stirred at 80°C overnight. After the reaction, the reaction mixture was concentrated under reduced pressure, and ethyl acetate was added. The obtained mixture was washed successively with water and saturated brine. The organic layer was concentrated under reduced pressure, and the residue was purified by silica gel chromatography to give the title compound (1.16 g, 5.45 mmol).
¹H-NMR (CDCl₃) δ 1.60 (3H, s), 2.57(3H, s), 3.21(6H, s), 7.28 (1H, d, J=5.1Hz), 8.53(1H, d, J=5.1Hz)

### Example 7: 4-(1,1-dimethoxy-ethyl)-2-methylsulfanyl-pyrimidine

Methylisothiourea sulfate (100 mg, 0.36 mmol) and sodium carbonate (76 mg, 0.71 mmol) were stirred in butyl acetate (1.5 ml) for 30 min, 1,4,4-trimethoxy-pent-1-en-3-one (120 mg, 0.69 mmol) was added thereto, and the mixture was stirred at 110°C for 5 hr. After the reaction, the reaction mixture was analyzed by HPLC under the same conditions as in Example 3, and production of the title compound (137 mg, 0.64 mmol) was confirmed.

### Example 8: 4-(1,1-dimethoxy-ethyl)-2-methylsulfanyl-pyrimidine

Methylisothiourea sulfate (61 mg, 0.22 mmol) and sodium carbonate (50 mg, 0.47 mmol) were stirred in methanol for 30 min, 1-ethoxy-4,4-dimethoxy-pent-1-en-3-one (79 mg, 0.42 mmol) was added thereto, and the mixture was stirred at 80°C for 2 hr. After the reaction, the reaction mixture was analyzed by HPLC under the same conditions as in Example 3, and production of the title compound (67.5mg, 0.32 mmol) was confirmed.

### Example 9: 4-(1,1-dimethoxy-ethyl)-2-methoxy-pyrimidine

Methoxyisourea hydrochloride (761 mg, 6.88 mmol) and sodium carbonate (791 mg, 7.46 mmol) were stirred in ethyl acetate (10 ml) for 30 min, 1,4,4-trimethoxy-pent-1-en-3-one (1.0 g, 5.74 mmol) was added thereto, and the mixture was stirred at 80°C overnight. After the reaction, the reaction mixture was concentrated under reduced pressure, and ethyl acetate was added. The obtained mixture was washed successively with water and saturated brine. The organic layer was concentrated under reduced pressure, and the residue was purified by silica gel chromatography to give the title compound (914 mg, 4.62 mmol).
¹H-NMR(CDCl₃) δ 1.60(3H, s), 3.22(6H, s), 3.99(3H, s), 7.22 (1H, d, J=5.0Hz), 8.48(1H,d, J=5.0Hz)

### Example 10: [4- (1,1-dimethoxy-ethyl) -pyrimidin-2-yl]methylamine

Methylguanidine hydrochloride (755 mg, 6.90 mmol) and sodium carbonate (790 mg, 7.45 mmol) were stirred in ethyl acetate (10 ml) for 30 min, 1,4,4-trimethoxy-pent-1-en-3-one (1.0 g, 5.74 mmol) was added thereto, and the mixture was stirred at 80°C overnight. After the reaction, the reaction mixture was concentrated under reduced pressure, and ethyl acetate was added. The obtained mixture was washed successively with water and saturated brine. The organic layer was concentrated under reduced pressure, and the residue was purified by silica gel chromatography to give the title compound (526 mg, 2.66 mmol).
¹H-NMR(CDC1₃) δ 1.56(3H, s), 3.01(3H, d, J=5.1Hz), 3.22(6H, s), 5.24 (1H, br), 6.84 (1H, d, J=5.1Hz), 8.33 (1H, d, J=5.1Hz)

### Example 11: 4-(1,1-dimethoxy-ethyl)-2-phenyl-pyrimidine

Benzamidine hydrochloride (1.10 g, 6.3 mmol) and sodium carbonate (0.73 g, 6.9 mmol) were stirred in ethyl acetate (10 ml) for 30 min, 1,4,4-trimethoxy-pent-1-en-3-one (1.0 g, 5.74 mmol) was added thereto, and the mixture was stirred at 80°C overnight. After the reaction, the reaction mixture was concentrated under reduced pressure, and ethyl acetate was added. The obtained mixture was washed successively with water and saturated brine. The organic layer was concentrated under reduced pressure and the residue was washed with hexane to give the title compound (1.40 g, 5.70 mmol).
¹H-NMR(CDC1₃) δ 1.71(3H, s), 3.27(6H, s), 7.46-7.59 (4H, m), 8.50-8.52(2H, m), 8.81(1H, d, J=5.1Hz)

### Example 12: 1-(2-methylsulfanyl-pyrimidin-4-yl)-ethanone

4-(1,1-Dimethoxy-ethyl)-2-methylsulfanyl-pyrimidine (500 mg, 2.33 mmol) was dissolved in ethyl acetate (7 ml), 1M hydrochloric acid (5 ml) was added, and the mixture was stirred for 1 hr. The mixture was partitioned, and the obtained organic layer was washed with saturated brine and concentrated under reduced pressure. Hexane (5 ml) was added to the concentrate to allow stirring for 3 hrs in an ice bath. The precipitate was filtrated, and dried under reduced pressure to give the title compound (254 mg, 1.52 mmol) as crystals. melting point 35°C
¹H-NMR(CDCl₃) δ 2.63(3H, s), 2.69(3H, s), 7.50(1H, d, J=4.9Hz), 8.73(1H, d, J=4.9Hz)

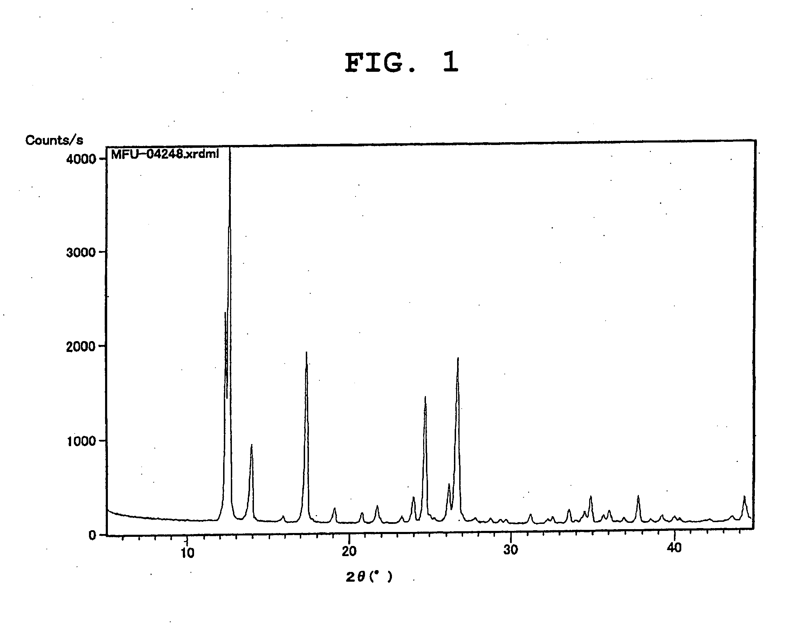

The powder X-ray diffraction pattern of the thus-obtained crystals was measured under the following conditions. As a result, characteristic peaks were found at diffraction angles (2θ±0.1°) of about 12.3°, about 12.6°, about 17.4°, about 24.7° and about 26.5°. The X-ray powder diffraction pattern is shown in Fig. 1.

For the measurement of powder X-ray diffraction, a powder X-ray diffraction apparatus X'Pert (manufactured by PANalytical) equipped with semiconductor array detector X'Celerator was used under the conditions of tube:Cu, tube electric current: 55 mA, tube voltage: 40 kV, sampling width:0.017°, scanning rate: 0.269°/sec, wavelength: 1.54056Å, measurement diffraction angle range (2θ): 5 - 45°C.

### Example 13: 1-(2-methylsulfanyl-pyrimidin-4-yl)-ethanone

4-(1,1-Dimethoxy-ethyl)-2-methylsulfanyl-pyrimidine (1.50, 7.0 mmol) was dissolved in ethyl acetate (5 mL), 2M hydrochloric acid (5 ml) was added thereto, and the mixture was stirred for 1 hr. The reaction mixture was partitioned, and the obtained organic layer was washed with saturated brine and concentrated under reduced pressure. Acetone (1.5 mL) was added to the residue, and water (12 mL) was added dropwise. The mixture was stirred overnight in an ice bath, and the precipitate was filtrated, and dried under reduced pressure to give the title compound (1.00 g, 5.95 mmol) as crystals. melting point 37°C

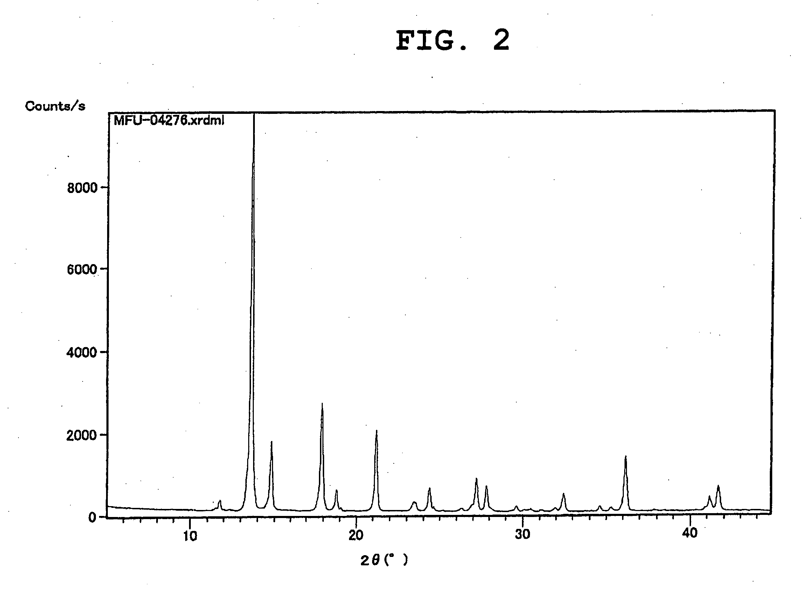

The powder X-ray diffraction pattern of the thus-obtained crystals was measured under the same conditions as in Example 12. As a result, characteristic peaks were found at diffraction angles (2θ±0.1°) of about 13.7°, about 14.8°, about 17.9°, about 21.2° and about 36.1°. The X-ray powder diffraction pattern is shown in Fig. 2.

### Example 14: 1-(2-methylsulfanyl-pyrimidin-4-yl)-ethanone

To 1-hydroxy-4,4₋dimethoxy-pent-1-en-3-one-sodium salt (10.38 g, 57.0 mmol) were added ethyl acetate (110 mL) and methyl sulfate (7.40 g, 58.7 mmol), and the mixture was stirred at 80°C overnight. The mixture was analyzed by HPLC under the same conditions as in the above-mentioned Example 3, and the reaction yield was confirmed to be 99%. The reaction mixture was cooled, the precipitate was filtered off under nitrogen, methylisothiourea sulfate (9.30 g, 33.4mmol) and sodium carbonate (7.65 g, 72.2 mmol) were added, and the mixture was stirred at 80°C overnight (cyclization yield: 90% by HPLC analysis under the same conditions as in the above-mentioned Example 3). After the reaction, the mixture was washed successively with water and 1M hydrochloric acid, and the solvent was evaporated. To the residue was added acetone (7 ml), then 1M hydrochloric acid (22 ml) was added, and the mixture was stirred at room temperature for 1 hr. The mixture was neutralized to pH 6 with an aqueous sodium hydroxide solution and stirred overnight in an ice bath. The precipitate was filtrated, and dried under reduced pressure to give the title compound (7.18 g, 42.7 mmol).

### Industrial Applicability

According to the method of the present invention, compound (I) can be produced in a high yield without coloring and the like using an economical and low toxic reagent.

Moreover, according to the present invention, crystals of 1-(2-methylsulfanylpyrimidin-4-yl)ethanone can be obtained. As a result, the quality management as a synthetic intermediate for a pharmaceutical product has become easier, and distribution of the compound has become easy.

This application is based on application No. 2005-027100 filed in Japan, the contents of which are incorporated hereinto by reference.

## Claims

1. A production method of a compound represented by the formula (III): 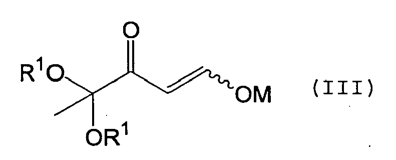 wherein R¹ is a lower alkyl group, or optionally bonded to each other to form an alkylene group optionally having substituent(s), M is an alkali metal and a wavy line is a trans-isomer, a cis-isomer or a mixture thereof, which comprises reacting a compound represented by the formula (II): 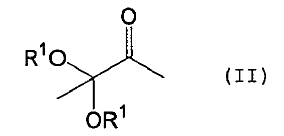 wherein R¹ is as defined above with a formate in the presence of an alkali metal alkoxide or an alkali metal hydride.

2. A production method of a compound represented by the formula (V) : 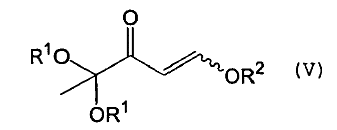 wherein R¹ is a lower alkyl group, or optionally bonded to each other to form an alkylene group optionally having substituent(s), R² is a lower alkyl group optionally having substituent(s) or an aralkyl group optionally having substituent(s) and a wavy line is a trans-isomer, a cis-isomer or a mixture thereof, which comprises reacting a compound represented by the formula (III): 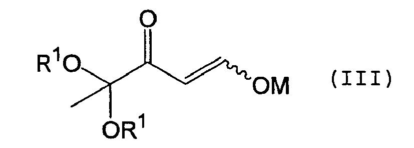 wherein M is an alkali metal and R¹ is as defined above with a compound represented by the formula (IVa): R²X¹ or the formula (IVb): (R²O)₂SO₂ (wherein X¹ is a halogen atom, and R² is as defined above.

3. A production method of a compound represented by the formula (VII) : 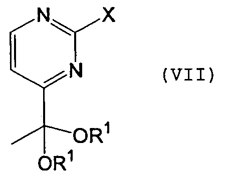 wherein R¹ is a lower alkyl group, or optionally bonded to each other to form an alkylene group optionally having substituent(s) and X is a hydrogen atom, a lower alkyl group optionally having substituent(s), an aryl group optionally having substituent(s), an aralkyl group optionally having substituent(s), -OR³, -SR³ or -NR³R⁴ (wherein R³ and R⁴ are the same or different and each is a hydrogen atom, a lower alkyl group optionally having substituent(s), an aralkyl group optionally having substituent(s) or an aryl group optionally having substituent(s), or R³ and R⁴ optionally form, together with the adjacent nitrogen atom, aliphatic hetero ring optionally having substituent(s)), which comprises reacting a compound represented by the formula (V) : 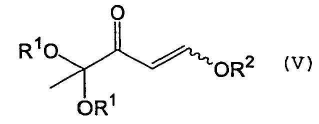 wherein R² is a lower alkyl group optionally having substituent(s) or an aralkyl group optionally having substituent(s), a wavy line is a trans-isomer, a cis-isomer or a mixture thereof and R¹ is as defined above with a compound represented by the formula (VI): 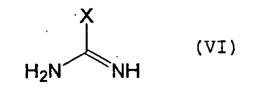 wherein X is as defined above or a salt thereof.

4. A production method of a compound represented by the formula (VII) : 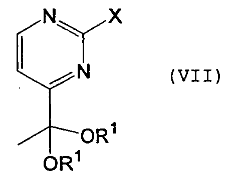 wherein R¹ is a lower alkyl group, or optionally bonded to each other to form an alkylene group optionally having substituent(s) and X is a hydrogen atom, a lower alkyl group optionally having substituent(s), an aryl group optionally having substituent(s), an aralkyl group optionally having substituent(s), -OR³ -SR³ or -NR³R⁴ (wherein R³ and R⁴ are the same or different and each is a hydrogen atom, a lower alkyl group optionally having substituent(s), an aralkyl group optionally having substituent(s) or an aryl group optionally having substituent(s), or R³ and R⁴ optionally form, together with the adjacent nitrogen atom, aliphatic hetero ring optionally having substituent(s)) or a salt thereof, which comprises the following steps (a) to (c);
(a) reacting a compound represented by the formula (II): 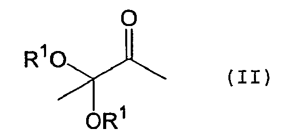 wherein R¹ is as defined above with a formate in the presence of an alkali metal alkoxide or an alkali metal hydride to give a compound represented by the formula (III): 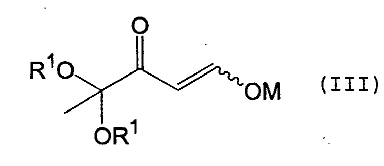 wherein M is an alkali metal and a wavy line is a trans-isomer, a cis-isomer or a mixture thereof and R¹ is as defined above;
(b) reacting the obtained compound represented by the formula (III) with a compound represented by the formula (IVa): R²X¹ or the formula (IVb): (R²O)₂SO₂ (wherein X¹ is a halogen atom and R² is a lower alkyl group optionally having substituent(s) or an aralkyl group optionally having substituent(s) to give a compound represented by the formula (V): 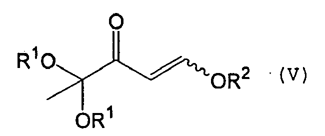 wherein each symbol and a wavy line are as defined above; and
(c) reacting the obtained compound represented by the formula (V) with a compound represented by the formula (VI): 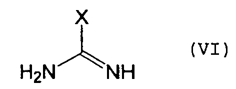
wherein X is as defined above or a salt thereof to give a compound represented by the formula (VII).

5. A production method of a compound represented by the formula (I) 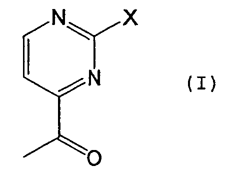 wherein X is a hydrogen atom, a lower alkyl group optionally having substituent(s), an aryl group optionally having substituent(s), an aralkyl group optionally having substituent(s), -OR³, -SR³ or -NR³R⁴ (wherein R³ and R⁴ are the same or different and each is a hydrogen atom, a lower alkyl group optionally having substituent(s), an aralkyl group optionally having substituent(s) or an aryl group optionally having substituent(s), or R³ and R⁴ optionally form, together with the adjacent nitrogen atom, aliphatic hetero ring optionally having substituent(s)) or a salt thereof, which comprises deprotecting the compound represented by the formula (VII) obtained by the method of claims 3 or 4.

6. The production method of any of claims 3 to 5, wherein X is a methyl group, a methylthio group, a methoxy group, a methylamino group or a phenyl group.

7. The production method of any of claims 1 to 5, wherein R¹ is a methyl group or an ethyl group.

8. The production method of any of claims 2 to 4, wherein R² is a methyl group, an ethyl group, an n-propyl group, an n-butyl group or a benzyl group.

9. A compound represented by the formula (V'): 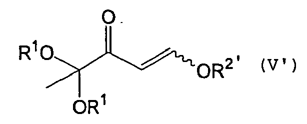 wherein R¹ is lower alkyl group, or may be bonded to each other to form an alkylene group optionally having substituent(s), R²' is an alkali metal, a lower alkyl group optionally having substituent(s) or an aralkyl group optionally having substituent(s), and a wavy line is a trans-isomer, a cis-isomer or a mixture thereof.

10. A production method of a crystal of 1-(2-methylsulfanylpyrimidin-4-yl)ethanone, which comprises crystallization from a mixed solvent of ethyl acetate and at least one solvent selected from a hydrocarbon solvent.

11. The production method of claim 10, wherein the crystallization is performed using a mixed solvent of hexane and ethyl acetate.

12. A production method of a crystal of 1-(2-methylsulfanylpyrimidin-4-yl)ethanone, which comprises crystallization from a mixed solvent of water and at least one solvent selected from acetone, methanol, ethanol and acetonitrile.

13. The production method of claim 12, wherein the crystallization is performed using a mixed solvent of acetone and water.

14. A crystal of 1-(2-methylsulfanylpyrimidin-4-yl)ethanone.

15. A crystal of 1-(2-methylsulfanylpyrimidin-4-yl)ethanone, showing a powder X-ray crystal diffraction pattern having characteristic peaks at diffraction angles (2θ±0.1°) of about 12.3°, about 12.6°, about 17.4°, about 24.7° and about 26.5°.

16. The crystal of claim 15, which is obtained by crystallization from a mixed solvent of hexane and ethyl acetate.

17. A crystal of 1-(2-methylsulfanylpyrimidin-4-yl)ethanone, showing a powder X-ray crystal diffraction pattern having characteristic peaks at diffraction angles (2θ±0.1°) of about 13.7°, about 14.8°, about 17.9°, about 21.2° and about 36.1°.

18. A crystal of claim 17, which is obtained by crystallization from a mixed solvent of acetone and water.
